# EUROPEAN PATENT SPECIFICATION

(11) **EP 1 319 713 B1**
(45) Date of publication and mention of the grant of the patent: **27.01.2010**
(21) Application number: 01965692.5
(22) Date of filing: 18.09.2001
(51) Int. Cl.: C12N 15/48, C12N 15/867, C07K 14/15

(54) **COMPLEX**
KOMPLEX
COMPLEXE

(30) Priority: 20.09.2000 JP 2000285857
(43) Date of publication of application: 18.06.2003
(73) Proprietor: TAKARA BIO INC., Otsu-shi, Shiga 520-2193 (JP)
(72) Inventor: CHONO, Hideto, Moriyama-shi, Shiga 524-0036 (JP); MATSUMURA, Hajime, Otsu-shi, Shiga 520-2134 (JP); UENO, Mitsuhiro, Kusatsu-shi, Shiga 525-0025 (JP); KATO, Ikunoshin, Uji-shi, Kyoto 611-0028 (JP)
(74) Representative: Schnappauf, Georg
(86) International application number: PCT/JP2001/008090
(87) International publication number: WO 2002/024916

(56) References cited:
- EP-A2- 0 611 822
- WO-A1-00/75342
- WO-A1-98/28417
- SHOJI-TANANKA A ET AL: "Gene Transfer using purified retroviral integrase" BIOCHEMICAL AND BIOPHYSICAL RESEARCH COMMUNICATIONS, ACADEMIC PRESS INC. ORLANDO, FL, US, vol. 203, 1994, pages 1756-1764, XP000929235 ISSN: 0006-291X
- MIZUARAI S ET AL: "INTEGRASE-MEDIATED NONVIRAL GENE TRANSFECTION WITH ENCHANCED INTEGRATION EFFICIENCY" JOURNAL OF BIOSCIENCE AND BIOENGINEERING, ELSEVIER, AMSTERDAM,, NL, vol. 88, no. 5, 1999, pages 461-467, XP002932667 ISSN: 1389-1723
- WEI SHUI-QING ET AL: "Footprints on the viral DNA ends in Moloney murine leukemia virus preintegration complexes reflect a specific association with integrase" PROCEEDINGS OF THE NATIONAL ACADEMY OF SCIENCES OF THE UNITED STATES OF AMERICA, vol. 95, no. 18, 1 September 1998 (1998-09-01), pages 10535-10540, XP002331096 ISSN: 0027-8424
- WEI SHUI-QING ET AL: "A large nucleoprotein assembly at the ends of the viral DNA mediates retroviral DNA integration" EMBO (EUROPEAN MOLECULAR BIOLOGY ORGANIZATION) JOURNAL, vol. 16, no. 24, 15 December 1997 (1997-12-15), pages 7511-7520, XP002331097 ISSN: 0261-4189
- HAJIME MATSUMURA ET AL.: 'Kumikae retrovirus kara no pre-integration complex(PIC) no chousei to idenshi dounyuu' DAI 24KAI NIPPON BUNSHI SEIBUTSU GAKKAI NENKAI, PROGRAM KOUEN YOUSHISHUU November 2001, page 853, 4P-699, XP002907455
- CHEREPANOV P. ET AL.: 'Activity of recombinant HIV-1 integrase on mini-HIV DNA' NUCLEIC ACIDS RES. vol. 27, no. 10, 1999, pages 2202 - 2210, XP002907456

### SEQUENCE LISTING

<110> Takara Shuzo Co., Ltd.
<120> Complexes
<130> 662777
<150> JP 2000-285857 <151> 2000-09-20
<160> 4
<210> 1 <211> 21 <212> DNA <213> Artificial Sequence
<220> <223> PCR primer to amplify a portion of GFP gene
<400> 1 caccctcgtg accaccctga c 21
<210> 2 <211> 24 <212> DNA <213> Artificial Sequence
<220> <223> PCR primer to amplify a portion of GFP gene
<400> 2 caccttgatg ccgttcttct gctt 24
<210> 3 <211> 24 <212> DNA <213> Artificial Sequence
<220> <223> PCR primer to amplify a portion of neo^{r} gene
<400> 3 gcttgatccg gctacctgcc catt 24
<210> 4 <211> 24 <212> DNA <213> Artificial Sequence
<220> <223> PCR primer to amplify a portion of neo^{r} gene
<400> 4 gccacagtcg atgaatccag aaaa 24

## Description

### Background Art

A method using a virus vector, a method transferring a naked DNA by means of endocytosis, electroporation or gene gun and the like are known as a method for transferring a gene Into a eukaryote. Virus vectors are utilized In a field of gene therapy for a wide range of studies including basic and clinical studies. For example, an adenovirus vector is suitable for transient expression of a gene of Interest In a target cell in large quantities. A retrovirus vector enables stable long-term expression due to its function of stable integration into a host chromosome. Thus, it is a hopeful vector in a field of gene therapy for genetic diseases and in a field of production of transgenic animals.

However, since gene transfer is mediated by viral infection according to a gene transfer method in which a virus vector is used, the tropism of the virus raises an issue. Specifically, one cannot transfer a gene into a cell that is not expressing a receptor for the virus on its surface. A pseudotype retrovirus vector has been developed in order to overcome this drawback. The pseudotype retrovirus vector utllizes an envelope glycoprotein from vesicular stomatitis virus, VSV-G, and can infect a wide range of hosts. This vector can be used to transfer a gene not only into mammals but also into fishes, birds, insects, amphibians and the like. Attempts have been made to apply the vector to production of transgenic animals. However, this vector has problems because one can transfer only one copy Into a target cell due to the nature of a retrovirus, and because it requires at least seven hours from infection to integration into a chromosome.

In case of gene transfer using a naked DNA, the DNA can be readily prepared in large quantities using Escherichia coll or the like, and a gene can be transferred, for example, by means of endocytosis mediated by calcium phosphate transfection or a cationic liposome transfection, electroporation, gene gun or the microinjection technique. In this case, some of the DNAs are integrated into chromosomes through recombination, although the efficiencies are quite low. Therefore, in most cases, these techniques are utilized only when transient gene expression is expected, and are unsuitable for stable long-term expression. Nevertheless, a technique in which a naked DNA is transferred by means of microinjection or the like is exclusively employed for production of transgenic organisms at present. Since integration of a gene into a chromosome is achieved only by accidental recombination as described above, the efflciency of production of a transgenic organism is quite low. Therefore, the production costs a lot and takes a lot of time under the present conditions.

### Objects of invention

The main object of the present invention is to provide a method for transducing a wide range of cells by transferring a gene regardless of host range utilizing a function of stable integration into a chromosome, which is characteristic of a retrovirus vector.
Thereby, it is possible to establish a technique by which a wide variety of non-human transgenic organisms can be produced and to develop a novel vector in a field of gene therapy.

### Summary of invention

As a result of intensive studies, the present Inventors have prepared a recombinant pre-integration complex (rPIC), transferred it into a target cell, and surprisingly found that the a gene contained in the rPIC is integrated into the chromosome of the target cell regardless of the tropism of the original virus. The rPIC is formed immediately before a recombinant retrovirus vector is integrated into a host chromosome.
The present invention is outlined as follows.
The present invention relates to an in vitro method for integrating a DNA into a chromosome of a cell, the method comprising:
transferring, into the cell, a recombinant pre-integration complex that comprises a double-stranded DNA containing two long terminal repeats derived from a retrovirus and the DNA to be integrated which is inserted between the long terminal repeats and has a nucleotide sequence that is of origin different from the retrovirus as well as a protein component which is derived from a retrovirus and has an activity of integrating a viral DNA into a host DNA,
   wherein the cell is not a human embryo or a human embryonic cell, and
   wherein the recombinant pre-integration complex is obtainable by a method consisting of:
   (a) infecting a cell which is arrested at the G1 phase with a cell cycle-regulating agent and is susceptible to a recombinant retrovirus with a recombinant retrovirus that contains the long terminal repeats and the nucleotide sequence that is of origin different from the retrovirus which is inserted between the long terminal repeats;
   (b) culturing the cell infected in step (a); and
   (c) extracting the recombinant pre-integration complex that accumulated in the cytoplasm and comprises a double-stranded DNA derived from the recombinant retrovirus and a protein component which is derived from the retrovirus from the cell cultured in step (b), by degrading the cell membrane with a detergent.
In one embodiment of the method according to the invention, the DNA is integrated into a chromosome of a eukaryotic cell.
In another embodiment, the DNA is integrated into a chromosome of a cultured cell derived from a eukaryotic cell.
In another embodiment, DNA is integrated into a chromosome of a non-human vertebrate fertilized egg.
In another embodiment, the DNA is integrated into a chromosome of a-non-human vertebrate germ line.

### Detailed Description of the invention

The present invention is described in detail below.

The complex used in the method of the present invention is a complex that comprises at least a double-stranded DNA containing two long terminal repeats (LTR) derived from a retrovirus and a DNA having a nucleotide sequence that is absent in the retrovirus (i.e., a DNA that is of origin different from the retrovirus) as well as a protein component derived from a retrovirus. The complex does not have a structure as a retrovirus. Specifically, the complex used in the method of the present invention is not infectious because it does not have an RNA genome and an envelope of a retrovirus. In one embodiment, the long terminal repeat derived from a retrovirus and the DNA having a nucleotide sequence that is absent in the retrovirus are derived from a recombinant retrovirus. By using the complex, a gene contained in the double-stranded DNA derived from a recombinant retrovirus, or the DNA having a nucleotide sequence that is absent In the retrovirus, in the complex can be transferred into a target cell. As used herein, a recombinant retrovirus refers to a retrovirus in which a nucleotide sequence of a naturally occurring retroviral genome is partially modified. For example, recombinant retroviruses include a retrovirus into which a foreign gene is inserted, and a retrovirus In which a gene on the viral genome or a portion thereof is subjected to deletion, substitution, Insertion or addition. There is no specific limitation concerning the recombinant retrovirus. It may be ecotropic or amphotropic, or of a pseudotype. For example, a commercially available recombinant retrovirus vector can be used.

There is no specific limitation concerning the DNA having a nucleotide sequence that is absent in the virus. Any DNA of which the transfer into a cell is desired may be used. For example, a DNA that contains a gene encoding a protein such as an enzyme or a growth factor, or a DNA that contains a gene encoding an antisense nucleic acid, a ribozyme or the like may be used.

The DNA may contain an appropriate marker gene which enables selection of a cell having a gene being transferred. For example, a drug resistance gene which confers resistance to antibiotics on a cell, a reporter gene which enables distinction of a cell having a gene being transferred based on an enzymatic activity or the like may be utilized as a marker gene.

According to the present invention, a gene may be contained in the double-stranded DNA in the complex used in the method of the present invention under control of an appropriate promoter such as an LTR promoter in a retrovirus vector or a foreign promoter. Furthermore, the double-stranded DNA may Include another regulatory element that cooperates with a promoter or a transcription initiation site (e.g., an enhancer sequence or a terminator sequence) in order to accomplish efficient transcription of a foreign gene. In addition, the double-stranded DNA may include an insulator sequence or the like in order to avoid chromosomal position effect. The foreign gene to be transferred may be a naturally occurring one or an artificially prepared one. Alternatively, it may be obtained by connecting DNA molecules of different origins by a known means such as ligation.

As used herein, a protein component derived from a retrovirus refers to one that has an activity of integrating a viral DNA into a host DNA.
The protein component derived from a retrovirus used according to the present invention may have deletion, substitution, insertion or addition in a naturally occurring protein component derived from a retrovirus as long as it has an activity of integrating a viral DNA into a host chromosome.

A retrovirus infects a dividing and growing cell, and its viral gene is integrated into a chromosome of a host cell. Integration of a gene into a chromosome by a retrovirus is achieved through a series of steps as follows: (1) attachment of a viral particle to a surface of a target cell via a receptor; (2) release of a core for invasion Into a cytoplasm; (3) synthesis of a cDNA by a reverse transcriptase In the core; (4) synthesis of a double-stranded DNA utilizing an RNase H activity and a DNA-dependent DNA synthesis activity of the enzyme; (5) formation of a pre-integration complex (PIC); (6) transport of the PIC Into a nucleus; and (7) stable integration into a host chromosome by action of an enzyme having an integration activity.

Transport of a PIC into a nucleus of a host through breakdown of a nuclear membrane of a host cell is required for integration of a gene into a chromosome. The transport is achieved by passage through the G2/M phase of the host cell. Thus, the PIC cannot be transported into a nucleus In a non-dividing cell, and integration of a gene into the chromosome does not occur. Therefore, if a cell infected with a retrovirus is arrested at the G1 phase, the PIC that cannot be transported into a nucleus is accumulated in a cytoplasm. One can prepare the accumulated PIC from the cytoplasm.

For example, the complex used in the method of the present invention can be preferably prepared as follows utilizing the above-mentioned mechanism.

A cell arrested at the G1 phase by treatment with a cell cycle-regulating agent such as aphidicolin is infected with a recombinant retrovirus. The infected cell is cultured. As used herein, culturing is not limited to incubation of a cell for increasing the number of the cell, but includes maintenance of a cell in a state of arrest cell cycle. Reverse transcription, synthesis of a double-stranded DNA and formation of a recombinant pre-integration complex (rPIC) proceed in a cytoplasm during a period of seven to eleven hours from infection. Since the cell cycle is arrested at the G1 phase, the produced rPIC is not integrated into a chromosome, but accumulated In the cytoplasm. The rPIC accumulated In the cytoplasm can be readily extracted by degrading a cell membrane with a detergent. The extracted rPIC can be used In the method of the present invention. Furthermore, the extracted rPIC can be purified by a known method such as centrifugation or chromatography. The thus obtained purified rPIC can be also used in the method of the present invention.

The cultured cell used for the preparation of the complex used in the method of of the present invention may be any cell that can be infected with the retrovirus to be used. For example, NIH/3T3 cell (ATCC CRL-1658), HT-1080 cell (ATCC CCL-121) or 293 cell (ATCC CRL-1573) can be used.

It is possible to efficiently infect a cell arrested at the G1 phase with a recombinant retrovirus by using an appropriate supplementary agent such as polybrene or a functional substance as described in WO 95/26200 or WO 97/18318. A fibronectin fragment can be used as such a functional substance. Particularly, RetroNectin (Takara Shuzo) can be preferably used.

The complex used in the method of the present invention may be used for gene transfer immediately after preparation. Alternatively, it may be stored by freezing and used after thawing upon use. Although there is no specific limitation concerning the cryopreservation method, the complex may be rapidly frozen using dry ice and then stored at -80°C, for example.

By transferring the complex prepared as described above into a target cell, a gene can be transferred into a chromosome of the target cell, or the target cell can be transduced.

There is no specific limitation concerning the method for transferring the complex into a target cell. For example, the complex can be transferred by a known method such as a method in which endocytosis (e.g., a cationic liposome method or a calcium phosphate method), or a perforation method such as electroporation, gene gun or microinjection is utilized. If the complex is transferred using a perforation method, a transduced cell can be efficiently obtained by culturing a target cell in the presence of a substance having a cell adhesion activity after transfer as described in WO 96/17073.

There is no specific limitation concerning the origin of a target cell used for the method of the present invention. A wide variety of eukaryotic cells can serve as targets without restrictions due to the tropism of the recombinant retrovirus from which the complex used in the method of the present invention is derived. For example, a cell from a eukaryote such as a mammal, a bird, a fish, an insect or a plant can be used as a target.

There is no specific limitation concerning the type of the target cell. All kinds of cells (including, for example, stem cells such as a hematopoietic stem cell and non-human embryonic stem cells non-human germ cells such as an egg and a sperm, and various somatic cells) can serve as targets.

The target cell may be a cultured cell. A non-human transgenic animal can be produced by using a fertilized egg or a germ line from a vertebrate as a target.

A conventional gene transfer using a retrovirus vector requires at least seven hours from infection with a virus to integration of a gene into a chromosome. This is because formation of a pre-integration complex in a cytoplasm requires time. The target cells divide during the time, resulting in a mosaic in which cells with the transferred gene and cells without the transferred gene are generated. This phenomenon has been a great problem about production of transgenic animals.

On the other hand, according to the gene transfer method of the present invention, a gene is integrated into a chromosome immediately after transfer. Therefore, such a mosaic is not formed. Thus, the method of the present invention enables efficient production of a transgenic organism.

Furthermore, according to a conventional gene transfer using a retrovirus vector, one can transfer only one copy of a gene in a target cell. On the other hand, plural copies of a gene can be transferred into a target cell by adjusting the amount of the complex to be transferred according to the method of the present invention.

According to the present invention, the drawback associated with a conventional gene transfer method using a retrovirus vector, i.e., restriction on host range, is overcome, and a gene can be transferred Into all eukaryotic cells. Specifically, since the conventional gene transfer using a retrovirus vector Involves infection via a receptor for the virus, host range is restricted depending on the presence of the receptor. On the other hand, the complex used in the method of the present invention can be transferred using a known method such as a method in which endocytosis is utilized, electroporation, gene gun or microinjection regardless of the presence of such a receptor. As a result, a gene can be transferred Into a wide variety of cells without restriction on host range. Furthermore, a gene is integrated Into a chromosome immediately after transfer Into a target cell of Interest according to the present invention.
Therefore, a mosaic which has been a problem associated with a conventional method for producing a transgenic organism is not formed. A non-human transgenic organism can be efficiently produced.

As described above, the present invention is an excellent technique which overcomes the drawbacks associated with conventional techniques, and Is excellently useful, for example, in that a remarkable therapeutic effect is expected when the present invention is applied to any gene therapy of which the effect has been restricted because of the limitation associated with the conventional methods.

### Examples

The following Examples illustrate the present invention in more detail, but are not to be construed to limit the scope thereof.

### Example 1

Preparation of recombinant pre-integration complex (rPIC)
1. Preparation of ecoGFP virus supernatant

A cell producing a ecoGFP virus was established by introducing a plasmid containing a gene for green fluorescence protein (GFP) and a neomycin-resistance gene into a packaging cell GP+E-86 [Journal of Virology, 62: 1120-1124 (1988)]. The cell was cultured in Dulbecco's modified Eagle's medium (DMEM, Sigma) supplemented with 10% fetal calf serum (Bio Whittaker) containing 50 units/ml of penicillin (Gibco) and 50 µg/ml of streptomycin (Gibco) at 37°C in the presence of 5% CO₂. The DMEM used in procedures as described hereinafter was contained 50 units/ml of penicillin and 50 µg/ml of streptomycin in all cases. A suspension of the ecoGFP virus was prepared as follows. The producer cell was grown to semi-confluence in a 10-cm plate. 7 ml of DMEM supplemented with 10% fetal calf serum was added thereto. After culturing for 24 hours at 32°C in the presence of 5% CO₂, the culture supernatant was filtered through a 0.45-micron filter (Millipore) to prepare a stock of a viral supernatant, which was stored at -80°C until use.

### 2. Determination of titer of viral supernatant

Titer of the viral supernatant was determined according to a standard method [Journal of Virology, 62: 1120-1124 (1988)] using NIH/3T3 cell (ATCC CRL-1658). Briefly, 2 ml of DMEM supplemented with 10% calf serum (Gibco) containing 5x10⁴ NIH/3T3 cells was added to each well of a 6-well tissue culture plate (IwakI Glass). The plate was incubated at 37°C overnight In the presence of 5% CO₂. The medium was removed by suction. 1 ml of a serial dilution of the viral supernatant and hexadimethrine bromide (polybrene, Aldrich) at a final concentration of 8 µg/ml was added to each well. The plate was incubated at 37°C for four hours. 1 ml of DMEM supplemented with 10% calf serum was further added thereto, and the plate was incubated for 20 hours. The medium was then exchanged for 2 ml of DMEM supplemented with 10% calf serum containing G418 (Gibco) at a final concentration of 0.5 mg/ml. The incubation was further continued. The medium containing G418 was exchanged at intervals of three to four days. After 10 to 12 days, grown G418-resistant colonies were stained with a 0.2% methylene blue solution in, methanol and the number was counted. The number of infectious virus particles in 1 ml of a supernatant (cfu/ml) as the titer of the virus was calculated by multiplying the number of colonies in a well by the dilution ratio of the viral supernatant. The titer of the suspension of the ecoGFP virus prepared as described above was 1x10⁷ cfu/ml.

### 3. Preparation of rPIC

2 x 10⁶ NIH/3T3 cells were cultured in a 10-cm plate containing 10 ml of DMEM supplemented with 10% calf serum for 24 hours at 37°C in the presence of 5% CO₂. The medium was exchanged for 7 ml of DMEM supplemented with 10% calf serum containing 2 µg/ml of aphidicolin (Wako Pure Chemical industries). The plate was incubated for additional 14 hours at 37°C in the presence of 5% CO₂. The medium was removed by suction fourteen hours after the addition of aphidicolin. A mixture prepared by mixing 2 ml of the suspension of the ecoGFP virus with 3 ml of DMEM supplemented with 10% calf serum, and adding aphidicolin and polybrene at final concentrations of 2 µg/ml and 8 µg/ml, respectively, was added to the NIH/3T3 cells to start infection with the virus at 32°C in the presence of 5% CO₂. The viral suspension was removed three hours after the start of infection, and the medium was exchanged for 7 ml of DMEM supplemented with 10% calf serum containing 2 µg/ml of aphidicolin. The plate was incubated for additional seven hours (for 10 hours from the start of viral Infection) at 32°C in the presence of 5% CO₂. During this period, reverse transcription and DNA synthesis reactions proceeded to form rPICs in the NIH/3T3 cells Infected with the virus. However, since the cell cycle of the NIH/3T3 cell was arrested at the G1 phase by the action of aphidicolin, the rPICs were accumulated in the cytoplasm without integrating the retroviral gene Into the chromosome.

The rPICs accumulated in the cytoplasm were extracted as follows. Briefly, cell were washed with 5 ml of PBS 10 hours after infection and dispersed using 2 ml of trypsin-EDTA (Bio Whittaker). 5 ml of DMEM supplemented with 10% calf serum was added thereto. Cells precipitated by centrifugation at 200 x g for five minutes were washed with 9 ml of a buffer A (10 mM Tris, 225 mM KCl, 5 mM MgCl₂, 1 mM DTT, 20 µg/ml aprotinin, pH 7.4). A precipitate obtained by centrifugation at 200 x g for five minutes was suspended in 250 µl of the buffer A. 1.25 µl of a 5% digitonin solution In DMSO was added to the cell suspension. A cytoplasmic fraction was extracted by allowing the mixture to stand at 25°C for five minutes. The extract was centrifuged at 1000 x g for three minutes. The supernatant was further centrifuged at 5000 x g for three minutes. 50 µl aliquots of the thus obtained supernatant as an rPIC suspension were dispensed and stored at -80°C.

### 4. Detection of double-stranded DNA

The GFP gene derived from the recombinant retrovirus was detected by a PCR using a dilution of the rPIC suspension obtained by extraction and cryopreserved as described in 3 above as a template and GFP gene-specific primers. A PCR reaction mixture prepared by mixing 0.5 µl of TaKaRa Taq (5 units/µl, Takara Shuzo), 5 µl of 10 x PCR buffer (Takara Shuzo), 8 µl of 1.25 mM dNTP mix, 1 µl of the template, 20 pmol of Primer 1 (SEQ ID N0:1), 20 pmol of Primer 2 (SEQ ID NO:2) and distilled water to a volume of 50 µl was overlaid with mineral oil, and subjected to heating at 94°C for 1 minute followed by a reaction of 30 cycles each cycle consisting of 94°C for 30 seconds, 59°C for 30 seconds and 72°C for 30 seconds. After reaction, 10 µl of the PCR reaction mixture was subjected to electrophoresis on 2% agarose gel to confirm an amplified fragment. A dilution of the rPIC or a DNA purified from the rPIC by phenol-chloroform extraction was used as a template. A fraction extracted from NIH/3T3 cells without viral infection in a similar manner was subjected to a PCR as a negative control. An expression plasmid vector for GFP was used as a positive control. As a result, amplification of a 316-bp fragment derived from the GFP gene was observed when the dilution of the rPIC or the DNA purified from the rPIC by phenol-chloroform extraction was used as a template. Therefore, it was shown that the rPIC prepared as described in 3 above contained the GFP gene derived from the recombinant virus.

### Example 2

### Transfer of rPIC into cultured cell

Mouse NIH/3T3 cells and human 293 cells (ATCC CRL-1573) were cultured at 37°C in the presence of 5% CO₂ using DMEM supplemented with 10% calf serum and DMEM supplemented with 10% fetal calf serum as growth media, respectively. The mouse NIH/3T3 cells and human 293 cells grown to confluence in 10-cm plates were detached from the plates by trypsinization. The respective cells were washed with serum-free DMEM and suspended in 700 µl of DMEM. 700 µl of the cell suspension and 100 µl of the rPlC prepared in Example 1 were placed in a 0.4-cm width electroporation cuvette (Bio-Rad) and subjected to electroporation using Gene Pulser II (Bio-Rad) at 250 V, 975 (µF and room temperature. A 1/10 volume for mouse NIH/3T3 cells or a 1/4 volume for human 293 cells was seeded into a 10-cm gelatin-coated plate (Iwaki Glass). The cells were cultured in the growth medium containing 0.5 mg/ml of G418 starting from the day after electroporation.

Cells were observed under a fluorescence microscope seven days after electroporation. Cells emitting green fluorescence were observed for both mouse NIH 3T3 cells and human 293 cells. Thus, It was shown that the transferred GFP gene was expressed In cells derived from both a mouse and a human.

Neomycin (G418)-resistant colonies were formed When the cultivation was further continued for two weeks after the electroporation In a G418 selection medium. The colonies were stained with methylene blue. 46 resistant colonies and 196 resistant colonies were observed for mouse NIH/3T3 cells and human 293 cells, respectively. Thus, it was shown that the transferred gene was stably expressed.

The above-mentioned results show that an rPIC prepared from an ecotropic virus which can infect only cells derived from mice could be used to stably transfer a gene into cells derived from humans into which such a gene could not have been transferred according to a conventional method, and that the rPIC Is a vector that can be used to stably transfer a gene regardless of host range.

### Example 3

Preparation of high-titer rPIC and assessment of integration efficiency of rPIC
1. Preparation of high-titer rPIC

[0078] A high-titer ecoGFP virus suspension was prepared by centrifuging 250 ml of the ecoGFP virus supernatant at a low speed (2900 x g) for 16 hours at 4°C, and suspending the precipitate In 5 ml of DMEM supplemented with 10% calf serum. The titer of the viral suspension was 4x10⁸ cfu/ml. The viral suspension was used to infect NIH/3T3 cells at M.O.I. of 200. High-titer rPICs were prepared as described in Example 2.

### 2. Assessment of integration efficiency of rPIC

Gene transfer into 293 cells was carried out by electroporation using the high-titer rPIC. As controls, gene transfers Into 293 cells were carried out a by electroporation or transfection using a plasmid vector containing the GFP gene and the neomycin-resistance gene. Three days after gene transfer, 293 cells expressing GFP were selectively recovered using a flow cytometer FACS Vantage (Becton Dickinson). The ratios of GFP-expressing cells of the recovered 293 cells were 94.3% (transfer of the rPIC), 94.3% (transfer of the plasmid vector by electroporation) and 98.8% (transfer of the plasmid vector by transfection), indicating that cells with the gene transiently transferred were selectively recovered. The 293 cells transiently expressing GFP were cultured In a G418+ medium or a G418- medium for two weeks. An efficiency of stable long-term gene expression as an Integration efficiency was calculated according to the following equation: [number of colonies in a plate with G418] + [number of colonies in a plate without G418]. The integration efficiencies were 98.7% (transfer of the rPIC), 7.5% (transfer of the plasmid vector by electroporation) and 1.4% (transfer of the plasmid vector by transfection), indicating that the rPIC Is a vector that results in an excellent integration efficiency.

### Example 4

### Transfer of rPIC into fertilized egg of medaka fish

A pair of mature medaka fish (Oryzias latipes, orange-red variety) consisting of a male and a female was bred in 3 L of tap water at 25°C under conditions of a light period for 14 hours and a dark period for 10 hours. They were fed with TetraFin (TetraWerKe) three to five times a day setting the amount of TotraFin such that it was consumed within three to five minutes, and then spawned. Eggs were collected immediately after spawning, separated each other using tweezers in a 8-ppm aqueous solution of methylene blue and washed with the aqueous solution. The high-titer rPIC prepared In Example 3 was purified on a gel filtration column Superose 6 (Amersham Pharmacia). 5 µl of a fraction containing the rPIC was placed In a microinjection pipette (prepared using a capillary tubing model GD-1, Narishige), and about 50 pl of the rPIC suspension was injected into each one-cell stage medaka fertilized egg under a microscope. 69 eggs were subjected to microinjection, placed in a 96-well plate such that each well contained one egg, and cultured in the 8-ppm aqueous solution of methylene blue at 25°C. Hatched fry eight days after spawning or thereafter were transferred one by one to a fish tank. Finally, a total of 47 fry hatched. 16 fry were sampled immediately after spawning, placed in 1.5-ml microtubes and stored at -20°C. A genomic DNA was extracted from a hatched fry as follows and subjected to detection of the neomycin-resistance gene by a PCR. Briefly, 100 µl of an extraction buffer (10 mM Tris, 100 mM NaCl, 10 mM EDTA, 39 mM DTT, 2% SDS, 50 µg/ml Proteinase K, pH 8) was added to each microtube containing one fry. The tube was Incubated at 60°C for four hours. The tube was stirred 30 and 60 minutes after the start of the incubation. 5 µg of RNase A was added per tube, and the tube was incubated at 37°C for an hour. A genomic DNA obtained after phenol-chloroform extraction and ethanol precipitation was dissolved In 50 µl of distilled water.

The transferred gene was detected by a PCR using 1 µl of the thus obtained genomic DNA solution as a template and primers specific for the neomycin-resistance gene. TaKaRa Taq (Takara Shuzo) was used for the reaction. A mixture of 0.5 µl of TaKaRa Taq, 5 µl of 10 x PCR buffer, 8 µl of dNTP mix, 1 µl of the diluted template, 20 pmol of Primer 3 (SEQ ID NO:3), 20 pmol of Primer 4 (SEQ ID NO:4) and distilled water to a volume of 50 µl was overlaid with mineral oil, and subjected to heating at 94°C for 1 minute followed by a reaction of 30 cycles each cycle consisting of 94°C for 30 seconds, 56°C for 30 seconds and 72°C for 30 seconds. 10 µl of the PCR reaction mixture was subjected to electrophoresis on 2% agarose gel to confirm an amplified fragment. As a result, the transferred gene was detected in 16 out of 16 medaka fishes Into which the rPIC was transferred by microinjection. Thus, the efficiency of the gene transfer was 100%. A fertilized egg of medaka fish Is at the one-cell stage for an hour after fertilization. It divides once in 35 minutes thereafter If a VSV-G retrovirus vector is used for a cell dividing so rapidly, a gene is integrated only at an advanced developmental stage because the reverse transcription process limits the rate. Furthermore, a plasmid vector is integrated into a host chromosome only by accidental recombination. On the other hand, since the rPIC is extracted immediately before integration Into the genome of NIH/3T3 cell, it is prepared in a state that results in a high integration efficiency. Therefore, integration into a genome at an early stage of cell division is expected. The results show that the rPIC is a vector that can be applied to a wide variety of subjects, for example, as a vector for producing a transgenic animal.

### industrial Applicability

The present invention provides a method for efficiently integrating a gene regardless of host range.
The method enables efficient production of a non-human transgenic organism.

### Sequence Listing Free Text

SEQ ID NO:1: PCR primer to amplify a portion of GFP gene.
SEQ ID NO:2: PCR primer to amplify a portion of GFP gene.
SEQ ID NO:3: PCR primer to amplify a portion of neo^{r} gene.
SEQ ID NO:4: PCR primer to amplify a portion of neo^{r} gene.

## Claims

1. An in vitro method for integrating a DNA into a chromosome of a cell, the method comprising:
transferring, into the cell, a recombinant pre-integration complex that comprises a double-stranded DNA containing two long terminal repeats derived from a retrovirus and the DNA to be integrated which is inserted between the long terminal repeats and has a nucleotide sequence that is of origin different from the retrovirus as well as a protein component which is derived from a retrovirus and has an activity of integrating a viral DNA into a host DNA,
wherein the cell is not a human embryo or a human embryonic cell, and
wherein the recombinant pre-integration complex is obtainable by a method consisting of :
(a) infecting a cell which is arrested at the G1 phase with a cell cycle-regulating agent and is susceptible to a recombinant retrovirus with a recombinant retrovirus that contains the long terminal repeats and the nucleotide sequence that is of origin different from the retrovirus which is inserted between the long terminal repeats;
(b) culturing the cell infected in step (a); and
(c) extracting the recombinant pre-integration complex that accumulated in the cytoplasm and comprises a double-stranded DNA derived from the recombinant retrovirus and a protein component which is derived from the retrovirus from the cell cultured in step (b), by degrading the cell membrane with a detergent.

2. The method according to claim 1, wherein the DNA is integrated into a chomosome of a eukaryotic cell.

3. The method according to claim 1, wherein the DNA is integrated into a chomosome of a cultured cell derived from a eukaryotic cell.

4. The method according to claim 1, wherein the DNA is integrated into a chomosome of a non-human vertebrate fertilized egg.

5. The method according to claim 1, wherein the DNA is integrated into a chomosome of a non-human vertebrate germ line.

## Patentansprüche

1. In vitro-Verfahren zum Integrieren einer DNA in ein Chromosom einer Zelle, wobei das Verfahren umfasst:
Transferieren eines rekombinanten Präintegrationskomplexes, der eine doppelsträngige DNA, die zwei lange terminale Wiederholungen, die von einem Retrovirus abgeleitet sind, und die zu integrierende DNA, die zwischen die langen terminalen Wiederholungen inseriert ist und eine Nukleotidsequenz aufweist, die eines anderen Ursprungs als das Retrovirus ist, enthält, sowie eine Proteinkomponente, die von einem Retrovirus abgeleitet ist und eine Aktivität aufweist, eine virale DNA in eine DNA eines Wirts zu integrieren, umfasst, in die Zelle,
wobei die Zelle kein menschlicher Embryo oder keine menschliche embryonale Zelle ist und der rekombinante Präintegrationskomplex durch ein Verfahren erhältlich ist, bestehend aus:
(a) Infizieren einer Zelle, die in der G1-Phase mit einem Zellzyklus-regulierenden Mittel arretiert ist und die für ein rekombinantes Retrovirus empfänglich ist, mit einem rekombinanten Retrovirus, das die langen terminalen Wiederholungen und die Nukleotidsequenz, die eines anderen Ursprungs als das Retrovirus ist und zwischen die langen terminalen Wiederholungen inseriert ist, enthält,
(b) Kultivieren der in Schritt (a) infizierten Zelle und
(c) Extrahieren des rekombinanten Präintegrationskomplexes, der in dem Cytoplasma angehäuft ist und eine von dem rekombinanten Retrovirus abgeleitete doppelsträngige DNA und eine Proteinkomponente, die von dem Retrovirus abgeleitet ist, umfasst, aus der in Schritt (b) kultivierten Zelle mittels Degradation der Zellmembran mit einem Detergenz.

2. Verfahren gemäß Anspruch 1, wobei die DNA in ein Chromosom einer eukaryontischen Zelle integriert wird.

3. Verfahren gemäß Anspruch 1, wobei die DNA in ein Chromosom einer kultivierten Zelle integriert wird, die von einer eukaryontischen Zelle abgeleitet ist.

4. Verfahren gemäß Anspruch 1, wobei die DNA in ein Chromosom eines befruchteten Eis eines nicht menschlichen Vertebraten integriert wird.

5. Verfahren gemäß Anspruch 1, wobei die DNA in ein Chromosom einer Keimlinie eines nicht menschlichen Vertebraten integriert wird.

## Revendications

1. Méthode in vitro destinée à intégrer un ADN dans un chromosome d'une cellule, la méthode comprenant :
le transfert, dans la cellule, d'un complexe de pré-intégration recombinant qui comprend un ADN double brin contenant deux longues séquences terminales répétées provenant d'un rétrovirus et l'ADN à intégrer qui est inséré entre les longues séquences terminales répétées et a une séquence nucléotidique qui est d'origine différente du rétrovirus ainsi qu'un composant protéique qui provient d'un rétrovirus et a une activité d'intégration d'un ADN viral dans un ADN hôte,
dans laquelle la cellule n'est pas une cellule d'embryon humain ou une cellule embryonnaire humaine, et dans laquelle le complexe de pré-intégration humain recombinant peut être obtenu par une méthode consistant à :
(a) infecter une cellule qui est arrêtée en phase G1 avec un agent de régulation du cycle cellulaire et est sensible à un rétrovirus recombinant avec un rétrovirus recombinant qui contient les longues séquences terminales répétées et la séquence nucléotidique qui est d'origine différente du rétrovirus qui est insérée entre les longues séquences terminales répétées ;
(b) cultiver la cellule infectée de l'étape (a) ; et
(c) extraire le complexe de pré-intégration recombinant qui s'est accumulé dans le cytoplasme et comprend un ADN double brin provenant du rétrovirus recombinant et un composant protéique qui provient du rétrovirus de la cellule cultivée à l'étape (b), en dégradant la membrane cellulaire avec un détergent.

2. Méthode selon la revendication 1, dans laquelle l'ADN est intégré dans un chromosome d'une cellule eucaryote.

3. Méthode selon la revendication 1, dans laquelle l'ADN est intégré dans un chromosome d'une cellule cultivée provenant d'une cellule eucaryote.

4. Méthode selon la revendication 1, dans laquelle l'ADN est intégré dans un chromosome d'un ovule fertilisé de vertébré non humain.

5. Méthode selon la revendication 1, dans laquelle l'ADN est intégré dans un chromosome d'une lignée germinale de vertébré non humain.
